# FASCICULE DE BREVET EUROPEEN

(11) **EP 1 463 467 B1**
(45) Date de publication et mention de la délivrance du brevet: **11.07.2007**
(21) Numéro de dépôt: 03712219.9
(22) Date de dépôt: 08.01.2003
(51) Int. Cl.: A61F 5/00

(54) **ANNEAU GASTRIQUE DE TRAITEMENT DE L OBESITE**
MAGENRING ZUR BEHANDLUNG VON FETTSUCHT
GASTRIC RING FOR THE TREATMENT OF OBESITY

(30) Priorité: 09.01.2002 FR 0200262
(43) Date de publication de la demande: 06.10.2004
(73) Titulaire: SOFRADIM PRODUCTION, 01600 Trévoux (FR)
(72) Inventeur: DULUCQ, Jean, Louis, F-33000 Bordeaux (FR); THERIN, Michel, F-69004 CROIX ROUSSE (FR)
(74) Mandataire: Brédeville, Odile Marie
(86) Numéro de dépôt international: PCT/FR2003/000039
(87) Numéro de publication internationale: WO 2003/057091

(56) Documents cités:
- EP-A- 0 611 561
- FR-A- 2 612 392
- US-A- 4 607 618

## Description

La présente invention concerne un anneau gastrique de traitement de l'obésité. Un tel anneau est également couramment dénommé "anneau de gastroplastie".

Il est connu de traiter l'obésité pathologique d'un patient en plaçant un anneau autour de l'estomac de ce patient, de manière à créer, sur la partie supérieure de l'estomac, une poche de dimensions restreintes et une ouverture d'écoulement des aliments ayant également des dimensions restreintes.

De tels anneaux sont bien connus dans leur principe, et les documents WO-A-86/04498 et EP-A-0 611 561 peuvent être cités en tant que documents illustrant des anneaux gastriques existants.

Certains des anneaux existants ont pour inconvénient d'être relativement agressifs pour la paroi de l'estomac, au point de provoquer des inflammations de cette paroi, voire, dans des cas extrêmes, des perforations de celle-ci. Cette agressivité résulte de l'aspect massif et rigide de cet anneau ainsi que, notamment, de la présence d'une poche gonflable située sur la face interne de l'anneau, cette poche gonflable permettant d'ajuster la surface de l'ouverture délimitée par l'anneau en exercant sur la paroi gastrique une pression dirigée radialement vers l'intérieur.

Les chambres implantables permettant, par voie percutanée, de gonfler ou de dégonfler de telles poches gonflables, ainsi que les tubulures reliant ces chambres et ces poches, ont pour inconvénient de présenter des risques de fuite, de migration et d'infection.

Les anneaux existants ont également pour inconvénient de présenter des risques de bascule ou de glissement appelé "slippage" et de nécessiter des ré-opérations lorsqu'il est nécessaire de les replacer ou de les retirer après une certaine durée de traitement.

La présente invention vise à remédier à l'ensemble de ces inconvénients des dispositifs existants.

L'anneau qu'elle concerne comprend, de manière connue en soi,
- une bande définissant une portion médiane propre à entourer la paroi de l'estomac et deux portions latérales mutuellement distantes propres à être assemblées l'une à l'autre pour maintenir la bande autour de l'estomac, et
- des moyens d'assemblage desdites portions latérales l'une à l'autre et de maintien de ces portions latérales en position d'assemblage.
   Selon l'invention, la bande est constituée par un assemblage, organisé ou non, de filaments ou fibres en un matériau biorésorbable ou biodégradable.
   Un tel assemblage permet à la bande d'avoir une déformabilité nettement plus élevée que celle des anneaux selon la technique antérieure, qui sont réalisés en une matière synthétique pleine et à souplesse limitée, et d'avoir un appui réparti sur la paroi de l'estomac. La bande de l'anneau selon l'invention est ainsi très peu agressive pour la paroi de l'estomac du patient.
   De plus et surtout, cet assemblage de filaments présente une structure poreuse lui permettant d'être colonisé par les cellules de la paroi de l'estomac jusqu'à former une fibrose autour de la bande. Cette fibrose engendre une contraction cicatricielle, qui rend inutile d'utiliser une poche gonflable pour ajuster la section de l'ouverture de l'anneau.
   L'intégration tissulaire progressive de la bande solidarise l'anneau à la paroi de l'estomac et permet une parfaite prévention de la migration ("slippage") de l'anneau et l'obtention d'une large surface de contact des tissus avec la bande, favorisant la résorption de cette dernière.
   Cette résorption lente des filaments constituant la bande permet une disparition progressive de cette bande, qui élimine la nécessité d'une ablation de l'anneau et donc d'avoir à réopérer le patient à cette fin.
   Les filaments ou fibres peuvent former des fils et ces fils peuvent être organisés, par exemple tissés ou tricotés. Ces fibres peuvent également être rassemblées à plat dans le désordre, par exemple agglomérées, sans tissage, notamment par entremêlement et/ou au moyen d'un liant.
   Les filaments peuvent être constitués par un polymère d'acide lactique ou polyglycolique, ou par un copolymère d'acide lactique ou polyglycolique.
   Ces matériaux présentent une cinétique de résorption sur plusieurs mois voire plusieurs années, apte à maintenir l'effet de la bande sur une période suffisante pour réduire de façon significative l'index de masse corporelle.
   De préférence, au moins la face de la bande destinée à venir au contact de la paroi de l'estomac comporte un revêtement lisse propre à séparer cette face et cette paroi au moins temporairement.
   Ce revêtement permet d'empêcher ou de différer le contact direct entre la bande et la paroi de l'estomac le temps que les phases les plus inflammatoires de la cicatrisation de cette paroi soit passées. L'intégration tissulaire précitée est donc dissociée dans le temps par rapport à la cicatrisation des lésions initiales générées par la mise en place de l'anneau, ce qui limite ainsi le risque d'érosion de la paroi gastrique.
   La structure poreuse de la bande favorise la liaison de ce revêtement avec cette bande.
   Ce revêtement peut être en un matériau biorésorbable, notamment un matériau collagénique réticulé.
   De préférence, lesdits moyens d'assemblage que comprend l'anneau selon l'invention sont conformés pour placer lesdites portions latérales de la bande selon une direction sensiblement radiale par rapport au cercle que forme la portion médiane de la bande lorsque l'anneau est mis en place sur l'estomac d'un patient, ces portions latérales faisant saillie vers l'extérieur de ce cercle.
   L'anneau gastrique selon l'invention n'implique donc pas de chevauchement de l'une de ces portions latérales l'une sur l'autre, un tel chevauchement conduisant, sur certains dispositifs de l'art antérieur, à créer une surépaisseur au niveau de la face radialement interne dudit cercle, qui est agressive pour la paroi de l'estomac.
   Selon une forme de réalisation préférée de l'invention, lesdits moyens d'assemblage incluent au moins une attache comprenant des parties mobiles propres à venir en prise avec lesdites portions latérales de la bande.
   La structure tricotée de la bande rend possible de réaliser la fixation desdites portions latérales au moyen d'une telle attache.
   Avantageusement, dans ce cas, lesdites parties mobiles sont déplaçables entre deux positions, à savoir une position de coulissement, dans laquelle l'attache peut coulisser le long desdites portions latérales, et une position de verrouillage desdites portions latérales, dans laquelle toute possibilité de coulissement de l'attache par rapport à ces portions latérales est empêchée.
   Le coulissement de l'attache le long desdites portions latérales permet d'ajuster le serrage de la bande autour de l'estomac.
   Lesdites portions latérales de la bande peuvent alors présenter une longueur supérieure à ce qui est nécessaire pour la prise d'appui de l'attache sur elles, cette longueur étant telle que l'attache peut être coulissée le long de ces portions latérales jusqu'à obtention de la section désirée pour l'ouverture délimitée par l'anneau, et être ensuite amenée en position de verrouillage de ces portions latérales.
   L'attache peut comprendre des moyens d'immobilisation desdites parties mobiles dans les positions respectives précitées de coulissement et de verrouillage, de préférence de type à encliquetage irréversible.
   Cette attache peut être biorésorbable ou non biorésorbable ; elle peut inclure un produit radio-opaque de type sulfate de barium lorsqu'elle n'est pas en un matériau radio-opaque par nature.
   Selon une possibilité, l'attache comprend deux parties mobiles dimensionnées pour entourer lesdites portions latérales de la bande et maintenir ces portions serrées l'une contre l'autre, ces parties mobiles étant reliées l'une à l'autre par l'une de leurs extrémités longitudinales, au moyen d'une charnière-film.
   Cette charnière-film peut alors être conformée de manière à maintenir normalement lesdites parties mobiles dans une position d'éloignement d'une partie mobile par rapport à l'autre, afin de faciliter l'engagement de l'attache sur lesdites portions latérales de la bande.
   L'une desdites parties mobiles peut comprendre au moins une nervure faisant saillie de sa face tournée vers l'autre partie mobile, cette autre partie mobile présentant alors une lumière dans laquelle la ou les nervures sont destinées à être engagées.
   Selon une autre possibilité, l'attache est en un matériau non élastiquement déformable, tel qu'un matériau métallique, et comprend des pattes déformables susceptibles, dans une position de déformation, de pénétrer dans le matériau qui forme lesdites portions latérales de la bande et d'assurer ainsi la fixation de ces portions latérales l'une par rapport à l'autre.
   Une telle attache en un matériau métallique a pour avantage d'être peu encombrante.
   Selon un exemple de réalisation de la bande de l'anneau conforme à l'invention, la bande comprend une nappe en fils monofilaments et trois nappes en fils multifilaments, et est tricotée sur un métier de type chaîne Rachel à quatre barres à passettes.
   Le nombre de fils de chacune des nappes est fonction de la largeur voulue pour la bande et du liage de chacune des nappes.
   Ainsi, dans l'exemple précité, pour un métier comprenant vingt-quatre aiguilles au pouce anglais, et pour une largeur de bande de 15 mm,
- une quatrième nappe est formée par neuf fils multifilaments ;
- une troisième nappe est formée par neuf fils monofilaments ;
- une deuxième nappe est formée par onze fils multifilaments ;
- une première nappe est formée par dix fils multifilaments,
et le liage de ces nappes est le suivant :
- pour ladite quatrième nappe : 55/00
- pour ladite troisième nappe : 00/55;
- pour ladite deuxième nappe : 10/01;
- pour ladite première nappe : 23/10,
(les nappes étant dénombrées selon la norme DIN ISO 10223).

Selon un autre exemple de réalisation de la bande de l'anneau conforme à l'invention, la bande comprend une nappe en fils monofilaments et une nappe en fils multifilaments et est tricotée sur un métier de type Rachel à deux barres à aiguilles et au moins deux barres à passettes ; l'une des barres à passettes est enfilée avec les fils multifilaments et exécute des mailles alternativement sur les aiguilles avant et arrière, et l'autre barre à passettes est enfilée avec les fils monofilaments et trame partiellement sous trois ou cinq aiguilles selon la rigidité de la bande désirée.

Le nombre de fils et le liage sur chacune des barres à passettes sont de préférence, dans cet exemple, les suivants:
- barre avant :
   vingt-six fils multifilaments ;
   liage : 1012/0121 ;
- barre arrière :
   vingt-deux fils monofilaments ;
   liage : 5500/0055.

La bande selon l'un ou l'autre des exemples ci-dessus a tout ou partie des caractéristiques suivantes :
a) absence d'allongement longitudinal significatif, ou allongement très limité (inférieur à 10 % sous 5daN), résultant de la réduction au strict minimum de la bride d'entre-mailles dans le cas d'un liage chaînette ;
b) absence de tuilage, résultant de ce liage chaînette et particulièrement de la trame partielle sous trois ou cinq aiguilles en fils monofilaments ;
c) platitude de la bande obtenue, résultant particulièrement de ladite trame avec les fils monofilaments, plus rigides que ceux utilisés pour le liage ;
d) obtention de bords longitudinaux non agressifs et non traumatisants, résultant du tricotage de la bande à la largeur désirée pour cette bande ; une bande obtenue par découpe dans un tricot de plus grande largeur que cette largeur de bande désirée aboutirait en effet à des bords longitudinaux agressifs et traumatisants pour la paroi de l'estomac, résultant de la trame en fils monofilaments, et ceci même par un découpage à chaud ;
e) non agressivité des faces principales de la bande, résultant du placement judicieux des fils monofilaments et des fils multifilaments, de la finesse de la jauge ainsi que du diamètre des fils utilisés ;
f) effilochage limité en cas de découpe, résultant du choix du liage de chacune des nappes ;
g) obtention d'un compromis optimal entre la porosité de la bande, pour une réhabitation tissulaire à coeur, la faible densité de fils, nécessaire pour une résorption peu inflammatoire, la résistance de la bande à l'allongement et l'atraumatisme de celle-ci, résultant du rapport optimisé de fils multifilaments et de fils monofilaments.

Pour sa bonne compréhension, l'invention est à nouveau décrite ci-dessous en référence au dessin schématique annexé représentant, à titre d'exemples non limitatifs, deux formes de réalisation possibles de l'anneau gastrique qu'elle concerne.
la figure 1 en est une vue en perspective, alors qu'il est mis en place sur l'estomac d'un patient ;
la figure 2 en est une vue en coupe selon la ligne II-II de la figure 1;
la figure 3 est une vue à échelle agrandie d'une attache qu'il comprend, selon une première forme de réalisation, en coupe selon la ligne III-III de la figure 4, et en position d'ouverture de cette attache ;
la figure 4 est une vue de l'attache en coupe selon la ligne IV-IV de la figure 3 ;
la figure 5 est une vue de l'attache similaire à la figure 3, dans une position de fermeture partielle de cette attache, permettant un coulissement de cette attache par rapport à des portions latérales d'une bande que comprend l'anneau;
la figure 6 est une vue de l'attache similaire à la figure 4, dans cette même position de fermeture partielle ;
la figure 7 est une vue de l'attache similaire à la figure 3, dans une position de fermeture totale de cette attache, interdisant toute possibilité de coulissement de l'attache par rapport aux portions latérales de ladite bande;
la figure 8 est une vue de l'attache similaire à la figure 4, dans cette même position de fermeture totale ;
la figure 9 est une vue de côté de l'attache selon une deuxième forme de réalisation ;
la figure 10 est une vue de cette attache selon une direction opposée à celle selon la figure 9 ;
la figure 11 est une vue de cette attache en coupe selon la ligne XI-XI de la figure 9, l'attache étant en position d'ouverture ;
la figure 12 est une vue de l'attache similaire à la figure 11, l'attache étant en position de fermeture partielle ; et
la figure 13 est une vue de l'attache similaire à la figure 11, l'attache étant en position de fermeture totale.

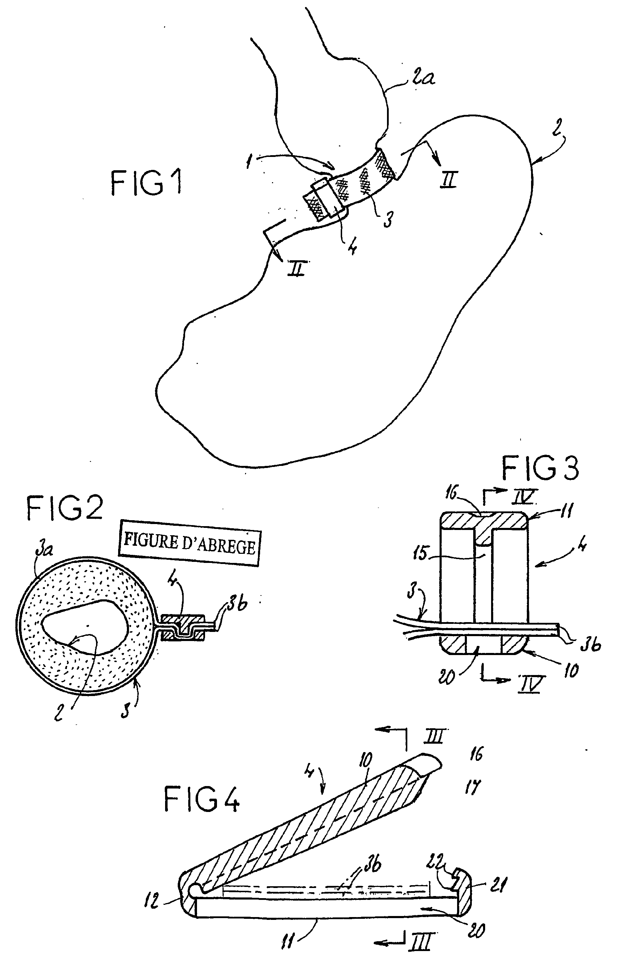

La figure 1 représente un anneau gastrique 1 de traitement de l'obésité pathologique d'un patient, mis en place sur l'estomac 2 de ce patient.

Cet anneau 1 comprend une bande 3 et une attache 4 propre à maintenir la bande 3 autour de l'estomac 2, afin de créer, sur la partie supérieure de l'estomac, une poche 2a de dimensions restreintes et une ouverture distale d'écoulement des aliments ayant également des dimensions restreintes.

Comme le montre la figure 2, la bande 3 définit une portion médiane 3a propre à entourer la paroi de l'estomac 2 selon un parcours circulaire et deux portions latérales 3b propres à être assemblées l'une à l'autre au moyen de l'attache 4, pour maintenir la bande 3 autour de l'estomac 2.

Les portions 3b présentent une longueur bien supérieure à ce qui est nécessaire pour la prise d'appui de l'attache 4 sur elles. Elles permettent ainsi une manipulation aisée de la bande, et notamment son passage rétrogastrique.

La bande 3 est constituée par un tricot de fils biorésorbables, par exemple formé par et assemblant une nappe en fils monofilaments et une nappe en fils multifilaments.

Cette bande 3 a été tricotée par exemple sur un métier de type Rachel à deux barres à aiguilles et au moins deux barres à passettes, l'une des barres à passettes étant enfilée avec les fils multifilaments et exécutant des mailles alternativement sur les aiguilles avant et arrière, et l'autre barre à passettes étant enfilée avec les fils monofilaments et tramant partiellement sous trois ou cinq aiguilles selon la rigidité de la bande 3 désirée.

La barre avant comprend vingt-six fils, et le liage est le suivant : 1012/0121 ; la barre arrière comprend vingt-deux fils, et le liage est le suivant : 5500/0055.

Les filaments ou fibres élémentaires des fils constitutifs du tricot sont constitués par un ou plusieurs matériaux biodégradables ou biorésorbables.

Par " biodégradable" ou "biorésorbable", on entend la propriété selon laquelle un matériau se dégrade in vivo par un mécanisme cellulaire, enzymatique ou microbien (cf par exemple dégradation du collagène par la collagènase), ou par un mécanisme physico-chimique (cf par exemple hydrolyse d'un polymère d'acide lactique).

Un tel matériau biorésorbable est de préférence choisi dans le groupe consistant en les polymères de p-dioxanone, les polyglycolides, les polyorthoesters, les polymères de triméthylène carbonate, les stéréocopolymères de l'acide L et D lactique, les homopolymères de l'acide L lactique, les copolymères de l'acide lactique et d'un comonomère compatible, tels que les dérivés d'alpha-hydroxy acides. De manière encore plus préférée, le matériau biorésorbable présente une polydispersité inférieure à 2.

A titre d'exemple préféré, le matériau biodégradable ou biorésorbable est un polymère d'acide lactique (PLA) ou polyglycolique (PGA), ou un copolymère d'acide lactique ou polyglycolique (PLA-PGA).

En référence aux figures 3 à 8, il apparaît que l'attache 4 comprend deux parties mobiles 10, 11 dimensionnées pour entourer les portions latérales 3b de la bande 3 et maintenir ces portions 3b serrées l'une contre l'autre selon une direction sensiblement radiale par rapport au cercle que forme la portion médiane 3a, comme le montre la figure 2, ces portions latérales 3b faisant saillie vers l'extérieur de ce cercle.

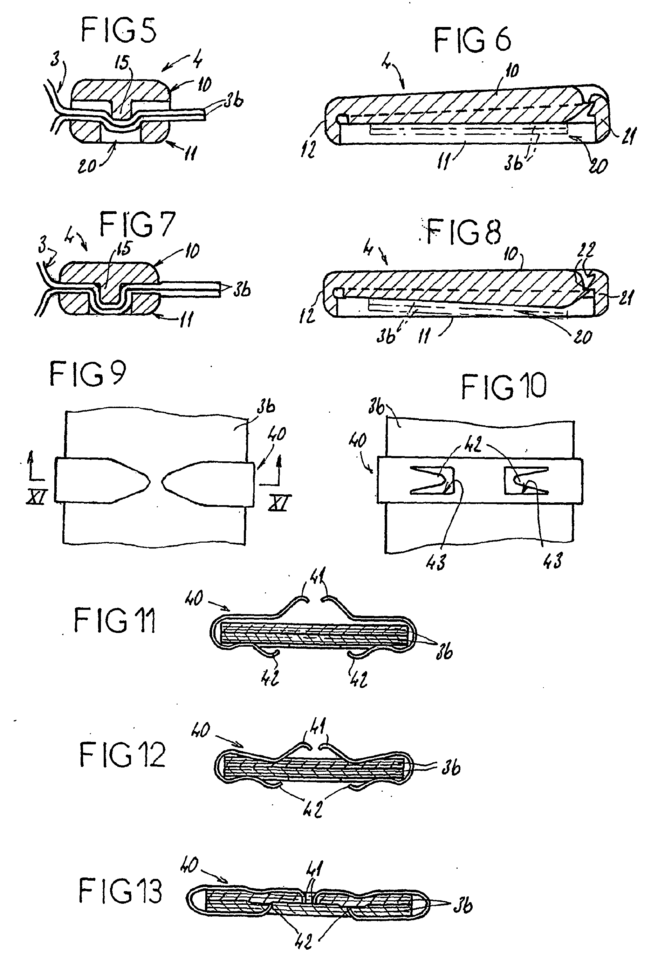

Les parties mobiles 10, 11 sont reliées l'une à l'autre par l'une de leurs extrémités longitudinales, au moyen d'une charnière-film 12. Cette charnière-film 12, à l'état non déformée, maintien les parties 10, 11 dans la position montrée sur les figures 3 et 4, dans laquelle la partie 10 est éloignée de la partie 11. Cet éloignement facilite d'engagement de l'attache 4 sur les portions 3b, particulièrement lorsque cette attache 4 est mise en place par des techniques peu invasives tel que coelioscopie ou laparoscopie. La charnière-film 12 peut être déformée pour permettre à la partie 10 de venir dans les positions de fermeture partielle et de fermeture totale de l'attache 4, représentées sur les figures 5 et 6, et 7 et 8, respectivement.

La partie 10 comprend une nervure 15 faisant saillie de sa face tournée vers la partie 11 et une encoche médiane 16 aménagée dans son bord latéral d'extrémité, du côté opposé à la charnière 12. La nervure 15 forme, au niveau du fond de l'encoche 16, un cran d'encliquetage 17, c'est-à-dire une saillie présentant une paroi inclinée du côté de la partie 11 et une paroi plane de verrouillage du côté opposé à cette partie 11.

La partie 11 présente une lumière centrale 20 dans laquelle la nervure 15 est destinée à être engagée, comme le montrent les figures 7 et 8. Du côté opposé à la charnière 12, cette partie 11 comprend une dent médiane 21 propre à être engagée dans l'encoche 16, qui est équipée de deux crans d'encliquetage étagés 22. Ces crans 22 sont propres à coopérer avec le cran 17 comme le montrent respectivement les figures 6 et 8.

Ainsi que cela se comprend en référence aux figures 3 à 8, ces crans 17 et 22 forment des moyens à encliquetage irréversible permettant de verrouiller lesdites parties mobiles 10, 11 l'une par rapport à l'autre selon deux positions, à savoir :
- une position de non-serrage des portions latérales 3b, montrée sur les figures 5 et 6, dans laquelle ces deux portions 3b peuvent coulisser avec frottements entre la nervure 15 et les portions de la partie 11 qui délimitent longitudinalement la lumière 20 ; et
- une position de verrouillage de ces portions latérales 3b, dans laquelle toute possibilité de coulissement de ces portions 3b est empêchée, du fait du serrage de ces portions 3b entre la nervure 15 et lesdites portions de la partie 11 qui délimitent longitudinalement la lumière 20.

L'attache 4 peut ainsi être mise en place sur les portions 3b alors qu'elle est en position d'ouverture, être amenée en position de non-serrage par simple pression de la partie 10 en direction de la partie 11 de manière à amener le cran 17 en prise avec le cran 22 supérieur, être coulissée le long de ces portions 3b jusqu'à obtention de la section désirée pour l'ouverture d'admission de nourriture dans l'estomac 2, et être amenée en position de verrouillage par simple pression de la partie 10 en direction de la partie 11 de manière à amener le cran 17 en prise avec le cran 22 inférieur. Les parties en excès des portions 3b peuvent être sectionnées.

Les figures 9 à 13 montrent une attache 40 selon une deuxième forme de réalisation, formée par pliage d'une bande métallique selon une forme de "C", de manière à délimiter un conduit dans lequel les portions 3b peuvent être engagées.

Cette attache 40 comprend, sur un côté, deux pattes recourbées 41 formées dans les extrémités de ladite bande métallique et, sur le côté opposé, deux pattes recourbées 42 individualisées par des découpes appropriées 43 de cette même bande métallique. Les pattes 41 sont susceptibles de pénétrer dans l'une des portions 3b tandis que les pattes 42 sont susceptibles de pénétrer dans l'autre portion 3b.

Comme le montre la figure 12, l'attache 40 peut être déformée en dehors des pattes 41, 42 pour l'obtention d'une possibilité de coulissement des portions 3b avec frottements dans l'attache 40.

La figure 13 montre que cette même attache 40 peut être déformée au niveau des pattes 41, 42 pour réaliser une pénétration de ces pattes 41, 42 dans les portions 3b afin de bloquer toute possibilité de coulissement de ces portions 3b dans l'attache 40.

Ainsi qu'il apparaît de ce qui précède, l'invention apporte une amélioration déterminante à la technique antérieure, en fournissant un anneau gastrique peu agressif pour la paroi de l'estomac, facile à mettre en place sur l'estomac du patient, et ne nécessitant pas une ablation en fin de traitement.

Cet anneau a particulièrement pour avantage d'éliminer la nécessité, après implantation, d'un réglage de la section de l'ouverture qu'il délimite, et donc d'éliminer la nécessité d'une chambre implantable.

De plus, la réhabitation tissulaire de la bande, grâce à la structure poreuse du tricot, induit une contraction fibreuse qui assure l'ajustement de cet anneau sur l'estomac.

La résorption lente de l'anneau laisse le temps au patient de perdre du poids et de se rééduquer tout en supprimant les risques de complications à long terme.

En outre, la disposition de la bande permise par l'attache permet une diminution maximale de l'agressivité du contact de cette bande avec la paroi gastrique.

## Revendications

1. - Anneau gastrique de traitement de l'obésité, comprenant :
- une bande (3) définissant une portion médiane (3a) propre à entourer la paroi de l'estomac et deux portions latérales (3b) propres à être assemblées l'une à l'autre pour maintenir la bande (3) autour de l'estomac (2), et
- des moyens d'assemblage (4, 40) desdites portions latérales (3b) l'une à l'autre et de maintien de ces portions latérales (3b) en position d'assemblage ;
anneau gastrique (1) **caractérisé en ce que** la bande (3) est constituée par un assemblage de filaments ou fibres en un matériau biorésorbable ou biodégradable.

2. - Anneau gastrique selon la revendication 1, **caractérisé en ce que** les filaments ou fibres forment des fils et **en ce que** ces fils sont tissés ou tricotés.

3. - Anneau gastrique selon la revendication 1, **caractérisé en ce que** des fibres sont agglomérées à plat, sans tissage ou tricotage, notamment par entremêlement et/ou au moyen d'un liant.

4. - Anneau gastrique selon l'une des revendications 1 à 3, **caractérisé en ce que** les filaments sont constitués par un polymère d'acide lactique ou polyglycolique, ou par un copolymère d'acide lactique ou polyglycolique.

5. - Anneau gastrique selon l'une des revendications 1 à 4, **caractérisé en ce qu'**au moins la face de la bande (3) destinée à venir au contact de la paroi de l'estomac (2) comporte un revêtement lisse propre à séparer cette face et cette paroi, au moins temporairement.

6. - Anneau gastrique selon la revendication 5, **caractérisé en ce que** ledit revêtement est en un matériau biorésorbable, notamment un matériau collagénique réticulé.

7. - Anneau gastrique selon l'une des revendications 1 à 6, **caractérisé en ce que** lesdits moyens d'assemblage (4, 40) sont conformés pour placer lesdites portions latérales (3b) de la bande (3) selon une direction sensiblement radiale par rapport au cercle que forme la portion médiane de la bande (3) lorsque l'anneau (1) est mis en place sur l'estomac (2) d'un patient, ces portions latérales (3b) faisant saillie vers l'extérieur de ce cercle.

8. - Anneau gastrique selon l'une des revendications 1 à 7, **caractérisé en ce que** lesdits moyens d'assemblage incluent au moins une attache (4, 40) comprenant des parties mobiles (10, 11 ; 41, 42) propres à venir en prise avec lesdites portions latérales (3b) de la bande (3).

9. - Anneau gastrique selon la revendication 8, **caractérisé en ce que** lesdites parties mobiles (10, 11 ; 41, 42) sont déplaçables entre deux positions, à savoir une position de coulissement, dans laquelle l'attache (4, 40) peut coulisser le long desdites portions latérales (3b), et une position de verrouillage desdites portions latérales (3b), dans laquelle toute possibilité de coulissement de l'attache (4, 40) par rapport à ces portions latérales (3b) est empêchée.

10. - Anneau gastrique selon la revendication 9, **caractérisé en ce que** lesdites portions latérales (3b) de la bande (3) présentent une longueur supérieure à ce qui est nécessaire pour la prise d'appui de l'attache (4, 40) sur elles, cette longueur étant telle que l'attache (4, 40) peut être coulissée le long de ces portions latérales (3b) jusqu'à obtention de la section désirée pour l'ouverture délimitée par l'anneau, et être ensuite amenée en position de verrouillage de ces portions latérales (3b).

11. - Anneau gastrique selon la revendication 8 ou la revendication 9, **caractérisé en ce que** l'attache (4, 40) comprend des moyens d'immobilisation (17, 22) desdites parties mobiles (10, 11 ; 41, 42) dans les positions respectives de coulissement et de verrouillage.

12. - Anneau gastrique selon la revendication 11, **caractérisé en ce que** lesdits moyens d'immobilisation (17, 22) sont de type à encliquetage irréversible.

13. - Anneau gastrique selon l'une des revendications 8 à 12, **caractérisé en ce que** l'attache (4) est réalisée en un matériau biorésorbable ou biodégradable.

14. - Anneau gastrique selon l'une des revendications 8 à 12, **caractérisé en ce que** l'attache (4, 40) est réalisée en un matériau non biorésorbable.

15. - Anneau gastrique selon l'une des revendications 8 à 14, **caractérisé en ce que** l'attache (4) comprend deux parties mobiles (10, 11) dimensionnées pour entourer les portions latérales (3b) de la bande (3) et maintenir ces portions serrées l'une contre l'autre, ces parties mobiles (10, 11) étant reliées l'une à l'autre par l'une de leurs extrémités longitudinales, au moyen d'une charnière-film (12).

16. - Anneau gastrique selon la revendication 15, **caractérisé en ce que** la charnière-film (12) est conformée de manière à maintenir normalement lesdites parties mobiles (10, 11) dans une position d'éloignement d'une partie mobile (10) par rapport à l'autre.

17. - Anneau gastrique selon la revendication 15 ou la revendication 16, **caractérisé en ce que** l'une desdites parties mobiles (10) comprend au moins une nervure (15) faisant saillie de sa face tournée vers l'autre partie mobile (11), et **en ce que** cette autre partie mobile (11) présente une lumière (20) dans laquelle la ou les nervures (15) sont destinées à être engagées.

18. - Anneau gastrique selon l'une des revendications 8 à 14, **caractérisé en ce que** l'attache (40) est en un matériau non élastiquement déformable tel qu'un matériau métallique et **en ce qu'**elle comprend des pattes déformables (41, 42) susceptibles, dans une position de déformation, de pénétrer dans le tricot qui forme lesdites portions latérales (3b) de la bande (3) et d'assurer ainsi la fixation de ces portions latérales (3b) l'une par rapport à l'autre.

19. - Anneau gastrique selon l'une des revendications 1 à 18, **caractérisé en ce que** ladite bande (3) comprend une nappe en fils monofilaments et trois nappes en fils multifilaments, et **en ce qu'**elle est obtenue par tricotage sur un métier de type chaîne Rachel à quatre barres à passettes.

20. - Anneau gastrique selon la revendication 19, **caractérisé en ce que** ladite bande (3) est telle qu'obtenue par tricotage de ladite nappe en fils monofilaments et desdites nappes en fils multifilaments, et **en ce que** cette bande (3) comprend, pour une largeur de bande (3) de 15 mm,
- une quatrième nappe formée par neuf fils multifilaments ;
- une troisième nappe formée par neuf fils monofilaments ;
- une deuxième nappe formée par onze fils multifilaments ;
- une première nappe formée par dix fils multifilaments,
et **en ce que** le liage de ces nappes est le suivant :
- pour ladite quatrième nappe : 55/00
- pour ladite troisième nappe : 00/55;
- pour ladite deuxième nappe : 10/01;
- pour ladite première nappe : 23/10,
les nappes étant dénombrées selon la norme DIN ISO 10223.

21. - Anneau gastrique selon l'une des revendications 1 à 18, **caractérisé en ce que** ladite bande (3) comprend une nappe en fils monofilaments et une nappe en fils multifilaments et est obtenue par tricotage sur un métier de type Rachel à deux barres à aiguilles et au moins deux barres à passettes, l'une des barres à passettes étant enfilée avec les fils multifilaments et exécutant des mailles alternativement sur les aiguilles avant et arrière, et l'autre barre à passettes étant enfilée avec les fils monofilaments et tramant partiellement sous trois ou cinq aiguilles selon la rigidité de la bande (3) désirée.

22. - Anneau gastrique selon la revendication 21, **caractérisé en ce que** le nombre de fils de ladite nappe en fils multifilaments est de 26 fils multifilaments, et **en ce que** ces fils sont placés sur la barre avant, avec un liage 1012/0121, tandis que le nombre de fils de la nappe en fils monofilaments est de vingt-deux fils monofilaments, engagés sur la barre arrière, le liage étant de 5500/0055.

## Claims

1. A gastric ring for treatment of obesity, comprising:
- a band (3) defining a central portion (3a) which is able to surround the wall of the stomach, and two lateral portions (3b) which can be connected to one another to hold the band (3) around the stomach (2), and
- means (4, 40) for connecting said lateral portions (3b) to one another and for maintaining these lateral portions (3b) in the connected position
said gastric ring (1) being **characterized in that** the band (3) consists of an assembly of filaments or fibers made of a bioabsorbable or biodegradable material,

2. The gastric ring as claimed in claim 1, **characterized in that** the filaments or fibers form yarns and **in that** these yarns are woven or knitted.

3. The gastric ring as claimed in claim 1, **characterized in that** fibers are agglomerated flat, without weaving or knitting, in particular by intermingling and/or by means of a binder.

4. The gastric ring as claimed in one of claims 1 through 3, **characterized in that** the filaments are made of a lactic or polyglycolic acid polymer or by a lactic or polyglycolic acid copolymer.

5. The gastric ring as claimed in one of claims 1 through 4, **characterized in that** at least the face of the band (3) intended to come into contact with the wall of the stomach (2) has a smooth coating for separating this face and this wall, at least temporarily.

6. The gastric ring as claimed in claim 5, **characterized in that** said coating s made of a bioabsorbable material, in particular a crosslinked collagen material

7. The gastric ring as claimed in one of claims 1 through 6, **characterized in that** said connection means (4, 40) are designed for placing said lateral portions (3b) of the band (3) in a substantially radial direction with respect to the circle formed by the central portion of the band (3) when the ring (1) is p aced on the stomach (2) of a patient, these lateral portions (3b) protruding outward from this circle.

8. The gastric ring as claimed in one of claims 1 through 7, **characterized in that** said connection means include at least one fastener (4, 40) comprising movable parts (10, 11; 41, 42) able to engage with said lateral portions (3b) of the band (3).

9. The gastric ring as claimed in claim 8, **characterized in that** said movable parts (10, 11; 41, 42) are displaceable between two positions, namely a position of sliding, in which the fastener (4, 40) can slide along said lateral portions (3b), and a position of locking of said lateral portions (3b), in which all possibility of sliding of the fastener (4, 40) relative to these lateral portions (3b) is prevented.

10. The gastric ring as claimed in claim 9, **characterized in that** said lateral portions (3b) of the band (3) have a length greater than that which is necessary for engagement of the fastener (4, 40) on these, this length being such that the fastener (4, 40) can be slid along these lateral portions (3b) until the des red cross section is obtained for the opening delimited by the ring, and can then be brought to the position of locking of these lateral portions (3b).

11. The gastric ring as claimed in claim 8 or claim 9, **characterized in that** he fastener (4, 40) comprises means (17, 22) for immobilizing said movable parts (10, 11; 41, 42) in the respective positions of sliding and of locking.

12. The gastric ring as claimed in claim 11, **characterized in that** said immobilizing means (17, 22) are of the irreversible snap-fit type.

13. The gastric ring as claimed in one of claims 8 through 12, **characterized in that** the fastener (4) is made of a bioabsorbable or biodegradable material.

14. The gastric ring as claimed in one of claims 8 through 12, **characterized in that** the fastener (4, 40) is made of a non-bioabsorbable material

15. The gastric ring as claimed in one of claims 8 through 14, **characterized in that** the fastener (4) comprises two movable parts (10, 11) which a e dimensioned to surround the lateral portions (3b) of the band (3) and to hold hese portions clamped against one another, these movable parts (10, 11) being connected to one another at one of their longitudinal ends, by means of a film hinge (12).

16. The gastric ring as claimed in claim 15, **characterized in that** the film hinge (12) is shaped in such a way as to hold said movable parts (10, 11) normally in a position in which one movable part (10) is at a distance from the other.

17. The gastric ring as claimed in claim 15 or claim 16, **characterized in that** one of said movable parts (10) comprises at least one rib (15) protruding from its face directed toward the other movable part (11), and **in that** this other movable part (11) has a slot (20) in which the rib or ribs (15) are intended to be engaged.

18. The gastric ring as claimed in one of claims 8 through 14, **characterized in that** the fastener (40) is made of a non-elastically deformable materia , for example a metal, and **in that** it comprises deformable tabs (41, 42) which, n a position of deformation, are able to penetrate into the knit forming said lateral portions (3b) of the band (3) and thereby ensure fixation of these lateral portions (3b) with respect to one another.

19. The gastric ring as claimed in one of claims 1 through 18, **characterized in that** said band (3) comprises a layer of monofilament yarns and three layers of multifilament yarns, and **in that** it is obtained by knitting on a Rasche loom with four guide bars.

20. The gastric ring as claimed in claim 19, **characterized in that** said band (3) is obtained by knitting of said layer of monofilament yarns and said layers of multifilament yarns, and **in that** this band (3) comprises, for a width ot band (3) of 15 mm,
- a fourth layer formed by nine multifilament yarns;
- a third layer formed by nine monofilament yarns ;
- a second layer formed by eleven multifilament yarns;
- a first layer formed by ten multifilament yarns,
and **in that** the binding of these layers is as follows:
- for said fourth layer: 55/00 ;
- for said third layer: 00/55;
- for said second layer: 10/01;
- for said first layer: 23/10,
the layers being numbered according to DIN ISO 10223.

21. The gastric ring as claimed in one of claims 1 through 18, **characterized in that** said band (3) comprises a layer of monofilament yarns and a layer of multifilament yarns and is obtained by knitting on a Raschel loom with two needle bars and at least two guide bars, one of the guide bars being threaded with the multifilament yarns and producing stitches alternately on the front and back needles, and the other guide bar being threaded with the monofilament yarns and running partially under three or five needles depending on the rigidity of the desired band (3).

22. The gastric ring as claimed in claim 21, **characterized in that** the number of yarns in said layer of multifilament yarns is 26 multifilament yarns, and in hat these yarns are placed on the front bar, with a binding 1012/0121, while the number of yarns in the layer of monofilament yarns is twenty-two monofilament yarns, engaged on the rear bar, the binding being 5500/0055.

## Patentansprüche

1. Magenring zur Behandlung von Übergewichtigkeit, mit:
- einem Band (3), das einen mittleren Abschnitt (3a) aufweist, der geeignet ist, die Magenwand zu umgeben, und zwei seitliche Abschnitte (3b) definiert, die geeignet sind, miteinander zusammengefügt zu werden, um das Band (3) um den Magen (2) herum zu halten, und
- Zusammenlügungsmitteln (4, 40) zum Zusammenfügen der seitlichen Abschnitte (3b) miteinander und zum Halten dieser seitlichen Abschnitte (3b) in der Zusammenfügungsstellung;
wobei der Magenring (1) **dadurch gekennzeichnet ist, dass** das Band (3) durch eine Anordnung von Filamenten oder Fasern aus einem bioresorbierbaren oder biodegradierbaren Material gebildet ist.

2. Magenring nach Anspruch 1, **dadurch gekennzeichnet, dass** die Filamente oder Fasern Fäden bilden, und dass diese Fäden gewebt oder gewirkt sind.

3. Magenring nach Anspruch 1, **dadurch gekennzeichnet, dass** die Fasern ohne Weben oder Wirken, insbesondere durch Vermengung und/oder mittels eines Bindemittels flach agglomeriert sind.

4. Magenring nach einem der Ansprüche 1 bis 3, **dadurch gekennzeichnet, dass** die Filamente aus einem Milchsäurepolymer oder einem Polyglycolpolymer oder aus einem Milchsäurecopolymer oder Polyglycolcopolymer gebildet sind.

5. Magenring nach einem der Ansprüche 1 bis 4, **dadurch gekennzeichnet, dass** zumindest die Seite des Bandes (3), die dazu bestimmt ist, mit der Wand des Magens (2) in Berührurg zu kommen, einen glatten Überzug aufweist, der geeignet ist, diese Seite und diese Wand zumindest zeitweilig zu trennen.

6. Magenring nach Anspruch 5, **dadurch gekennzeichnet, dass** der Überzug aus einem bioresorbierbaren Material, insbesondere einem vernetzten Collagenmaterial besteht.

7. Magenring nach einem der Ansprüche 1 bis 6, **dadurch gekennzeichnet, dass** die Zusammenfügungsmittel (4, 40) dazu ausgebildet sind, die seitlichen Abschnitte (3b) des Bandes 3) in einer bezüglich dem Kreis, den der mittlere Abschnitt des Bandes (3) bildet, wenn der Ring (1) am Magen (2) eines Patienten an Ort und Stelle gebracht ist, etwa radialen Richtung anzuordnen, wobei die seitlichen Abschnitte (3b) von dem Kreis nach außen vorspringen.

8. Magenring nach einem der Ansprüche 1 bis 7, **dadurch gekennzeichnet, dass** die Zusammenfügungsmittel zumindest eine Befestigung (4, 40) umfassen, die bewegliche Teile (10. 1 ; 41, 42) aufweist, die geeignet sind, mit den seitlichen Abschnitten (3b) des Bandes (3) in Eingriff zu kommen.

9. Magenring nach Anspruch 8, **dadurch gekennzeichnet, dass** die beweglichen Teile (10, 11; 41, 42) zwischen zwei Stellungen bewegbar sind, nämlich einer Verschiebestellung, in der die Befestigung (4, 40) entlang der seitlichen Abschnitte (3b) verschoben werden kann, und einer Verriegelungsstellung zur Verriegelung der seitlichen Abschnitte (3b), in der jede Möglichkeit einer Verschiebung der Befestigung (4, 40) bezüglich der seitlichen Abschnitte (3b) verhindert ist.

10. Magenring nach Anspruch 9, **dadurch gekennzeichnet, dass** die seitlichen Abschnitte 3b) des Bandes (3) eine Länge aufweisen, die größer ist als diejenige, die notwendig ist, um die Befestigung (4, 40) an ihnen anzulegen, wobei diese Länge derart ist, dass die Befestigung (4, 40) entlang der seitlichen Abschnitte (3b) verschoben werden kann, bis der für die Öffnung, die durch den Ring begrenzt wird, gewünschte Querschnitt erhalten ist, und anschließend in die Verriegelungsstellung zur Verriegelung der seitlichen Abschnitte (3b) gebracht werden kann.

11. Magenring nach Anspruch 8 oder Anspruch 9, **dadurch gekennzeichnet, dass** die Befestigung (4, 40) Festlegungsmittel (14, 22) zum Festlegen der beweglichen Teile (10, 1; 41, 42) in der jeweiligen Verschiebestellung und Verriegelungsstellung aufweist.

12. Magenring nach Anspruch 11, **dadurch gekennzeichnet, dass** die Festlegungsmittel (14, 22) von der Art einer irreversiblen Verrastung sind.

13. Magenring nach einem der Anspruche 8 bis 12, **dadurch gekennzeichnet, dass** die Befestigung (4) aus einem bioresorbierbaren oder biodegradierbaren Material gefertigt, ist

14. Magenring nach einem der Ansprüche 8 bis 12, **dadurch gekennzeichnet, dass** die Befestigung (4, 40) aus einem nicht bioresorbierbaren Material gefertigt ist.

15. Magenring nach einem der Ansprüche 8 bis 14, **dadurch gekennzeichnet, dass** die Befestigung (4) zwei bewegliche Teile (10, 11) aufweist, die so bemessen sind, dass sie die seitlichen Abschnitte (3b) des Bandes (3) umgeben und diese Abschnitte gegeneinander gedrückt halten, wobei die beweglichen Teile (10, 11) mit einem ihrer Längsenden mittels eines Filmscharniers (12) miteinander verbunden sind.

16. Magenring nach Anspruch 15, **dadurch gekennzeichnet, dass** das Filmscharnier (12) derart ausgebildet ist, dass es die beweglichen Tile (10, 11) normalerweise in einer Abstandsstellung des einen beweglichen Teils (10) bezüglich des anderen hält.

17. Magenring nach Anspruch 15 oder Anspruch 16, **dadurch gekennzeichnet, dass** eines der beweglichen Teile (10) zumindest eine Rippe (15) aufweist, die von seiner dem andere n beweglichen Teil (11) zugewandten Seite vorspringt, und dass das andere bewegliche Teil (11) eine Öffnung (20) aufweist, in der die Rippe oder die Rippen (15) eingreifen können.

18. Magenring nach einem der Ansprüche 8 bis 14, **dadurch gekennzeichnet, dass** die Befestigung (40) aus einen, nicht elastisch verformbaren Material, wie aus einem metallischer Material, besteht und dass sie verformbare Leisten (41, 42) aufweist, die in der Lage sind, in einer Verformungsstellung in das Gewirk, das die seitlichen Abschnitte (3b) des Bandes (3) bildet, einzudringen und so die Fixierung dieser seitlichen Abschnitte (3b) relativ zueinander zu gewährleisten.

19. Magenring nach einem der Ansprüche 1 bis 18, **dadurch gekennzeichnet, dass** das Band (3) eine Fadenschar aus Monofilamentfäden und drei Fadenscharen aus Multifilamentfäden aufweist, und dass es durch Wirken auf einer Kettwirkmaschine vom Typ Rachel mit vier Legebarren gefertigt ist.

20. Magenring nach Anspruch 19, **dadurch gekennzeichnet, dass** das Band (3) durch Wirken der Fadenschar aus Monofilamentlagen und den Fadenscharen aus Mulitfilamentfäden gefertigt ist, und dass das Band (3) bei einer Breite des Bandes (3) von 15 mm aufweist:
- eine vierte Fadenschar, die aus neun Multifilamentfäden gebildet ist;
- eine dritte Fadenschar, die aus neun Monofilamentfäden gebildet ist;
- eine zweite Fadenschar, die aus elf Multifilamentfäden gebildet ist;
- eine erste Fadenschar, die aus zehn Multifilamentfäden gebildet ist, und
dass die Bindung dieser Fadenscharen wie folgt ist :
- für die vierte Fadenschar: 55/00;
für die dritte Fadenschar : 00/55;
- für die zweite Fadenschar : 10/01;
- für die erste Fadenschar: 23/10,
wobei die Fadenscharen gemäß der Form DIN ISO 10223 gezählt sind.

21. Magenring nach einem der Ansprüche 1 bis 18, **dadurch gekennzeichnet, dass** das Band (3) eine Fadenschar aus Monofilamentfäden und eine Fadenschar aus Multifilamentfäden aufweist und durch Wirken auf einer Wirkmaschine vom Typ Rachel mit zwei Nadelbarren und zumindest zwei Legebarren gefertigt ist, wobei der eine der Legebarren nit den Multifilamentfäden aufgefädelt ist und abwechselnd auf den vorderen und hinterer Nadeln Maschen anfertigt, und der andere der Legebarren mit den Monofilamentfäden aufgefädelt ist und teilweise unter drei oder fünf Nadeln entsprechend der gewünschten Steifigkeit des Bandes (3) einschießt.

22. Magenring nach Anspruch 21, **dadurch gekennzeichnet, dass** die Anzahl an Fäden der Fadenschar aus Multifilamentfäden 26 Multifilamentfäden beträgt, und dass diese Fäder auf den vorderen Barren mit einer Bindung 1012/0121 angeordnet sind, während die Anzahl an Fäden der Fadenschar aus Monofilamentfäden 22 Monofilamentfäden beträgt, die mit dem hinteren Barren in Eingriff stehen, wobei die Bindung 5500/0055 ist.
